(19) Europäisches Patentamt

European Patent Office

Office européen des brevets

(11) **EP 1 034 464 B1**

(12) **EUROPÄISCHE PATENTSCHRIFT**

(45) Veröffentlichungstag und Bekanntmachung des Hinweises auf die Patenterteilung:
**12.06.2002 Patentblatt 2002/24**

(21) Anmeldenummer: **98963325.0**

(22) Anmeldetag: **05.11.1998**

(51) Int Cl.⁷: $G05B\ 23/02$, $A61N\ 1/36$

(86) Internationale Anmeldenummer:
**PCT/DE98/03229**

(87) Internationale Veröffentlichungsnummer:
**WO 99/24884 (20.05.1999 Gazette 1999/20)**

(54) **ANORDNUNG ZUR VORHERSAGE EINER ABNORMALITÄT EINES SYSTEMS UND ZUR DURCHFÜHRUNG EINER DER ABNORMALITÄT ENTGEGENWIRKENDEN AKTION**

ARRANGEMENT FOR PREDICTING AN ABNORMALITY OF A SYSTEM AND FOR CARRYING OUT AN ACTION WHICH COUNTERACTS THE ABNORMALITY

DISPOSITIF SERVANT A PREDIRE UNE ANOMALIE DANS UN SYSTEME ET A PRENDRE DES MESURES PERMETTANT DE CORRIGER CETTE ANOMALIE

(84) Benannte Vertragsstaaten:
**DE FR GB IT**

(30) Priorität: **07.11.1997 DE 19749373**

(43) Veröffentlichungstag der Anmeldung:
**13.09.2000 Patentblatt 2000/37**

(73) Patentinhaber: **SIEMENS AKTIENGESELLSCHAFT 80333 München (DE)**

(72) Erfinder:
• **DECO, Gustavo D-85579 Neubiberg (DE)**
• **DUBE', Louis-J. Sillery, G1T 2M4 (CA)**

(56) Entgegenhaltungen:
WO-A-96/36860      DE-C- 4 337 110
US-A- 4 889 526      US-A- 5 251 626

• **L.MACHADO ET AL: "SEQUENTIAL VERSUS STANDARD NEURAL NETORKS OR PATTERN RECOGNITION: AN EXAMPLE USING THE DOMAIN OF CORONARY HEART DISEASE" COMPUTERS IN BIOLOGY AND MEDECINE, Bd. 27, Nr. 4, Juli 1997, Seiten 267-281, XP002097626 UK**
• **D.HOYER. ET AL: "GRUNDLAGEN UND ERFAHRUNGEN ZUR MODELLIERUNG CHAOTISCHER ATTRAKTOREN DER HERZFREQUENZFLUKTUATIONEN MIT KUENSTLICHEN NEURONALEN NETZEN" BIOMEDIZINISCHE TECHNIK, Bd. 40, Nr. 7-8, Juli 1995, Seiten 190-194, XP002097627 germany**

EP 1 034 464 B1

**Beschreibung**

Die Erfindung betrifft eine Anordnung zur Vorhersage eines abnormalen Zustands eines Systems und zur Durchführung einer dem abnormalen Zustand entgegenwirkenden Aktion.

Die Bestimmung eines Informationsflusses eines Systems ist aus [1] und/oder [2] bekannt.

Der Informationsfluß kennzeichnet einen Verlust von Information in einem dynamischen System und beschreibt abklingende statistische Abhängigkeiten zwischen der gesamten Vergangenheit und einem Zeitpunkt, der p Schritte in der Zukunft liegt, als eine Funktion von p. Der Nutzen des Informationsflusses besteht u.a. darin, daß ein dynamisches Verhalten eines komplexen Systems klassifiziert werden kann, was dazu führt, daß ein geeignetes parametrisiertes Modell gefunden wird, das eine Modellierung von Daten des komplexen dynamischen Systems ermöglicht.

Ein neuronales Netz und das Training eines neuronalen Netzes sind bekannt aus [3].

Aus [6] und [7] sind Verfahren zur Erkennung von Unregelmäßigkeiten in einem Herzschlag unter Verwendung von neuronalen Netzen bekannt.

Die Aufgabe der Erfindung besteht darin, eine Anordnung anzugeben, die zum einen eine Vorhersage einer Abnormalität eines Systems ermöglicht und eine der Abnormalität entgegenwirkende Aktion durchführt.

Diese Aufgabe wird gemäß den Merkmalen des Patentanspruchs 1 gelöst.

Erfindungsgemäß angegeben wird eine Anordnung zur Vorhersage einer Abnormalität eines Systems und zur Durchführung einer der Abnormalität entgegenwirkenden Aktion. Darin ist ein Meßdatenaufnehmer vorgesehen, der Meßdaten des Systems ermittelt. Eine Prozessoreinheit ist derart eingerichtet, daß folgende Schritte durchgeführt werden:

(1) Anhand der Meßdaten wird ein neuronales Netz trainiert;
(2) der Informationsfluß des Systems wird verwendet, um eine Vorhersage über zu erwartende Meßdaten zu treffen;
(3) falls die Vorhersage anzeigt, daß die Abnormalität des Systems zu erwarten ist, wird die Aktion durchgeführt;

Abhängig von einer jeweiligen Anwendung ist dabei ein Aktor vorgesehen, der die Aktion ausführt.

Ziel der Erfindung ist eine systematische Annäherung an das allgemeine Problem und eine daraus abgeleitete Lösung dieses allgemeinen Problems der Bestimmung einer Größe (nachfolgend Vorhersagegröße genannt), die sich zur Vorhersage dynamischer Ereignisse eines Systems eignet. Die Früherkennung eines Musters, das einen Angriff auf ein "normales" Verhalten des Systems darstellt, ist von großer Bedeutung, wie u.a. nachfolgende Anwendungsbeispiele belegen.

Die angewandte Strategie unterteilt sich in drei Schritte:

1. Es werden die dynamisch kennzeichnenden Merkmale des Systems extrahiert und adaptiv gelernt (trainiert). In dieser dynamischen Lernphase sollte die Maßnahme, um die Dynamik des Systems zu erlernen, ausreichend allgemein sein, um stationärem gleichwie nichtstationärem Verhalten zu entsprechen. Die dynamische Lernphase wird auch dazu benutzt, um einen Normalzustand des Systems abzugrenzen gegen einen abnormalen Zustand (Abnormalität).

2. Es wird mindestens eine Variable (Vorhersagegröße) bestimmt, mit der es gelingt, die Abnormalität zu beschreiben.

3. Sobald ein Eintreten der Abnormalität angezeigt wird, wird die Information der bevorstehenden Abnormalität benutzt, um über einen Aktor, dessen Zweck es ist, das dynamische System wieder in den Normalzustand zu versetzen, der bevorstehenden Abnormalität entgegenzuwirken. Dabei ist zu berücksichtigen, daß der Normalzustand im Laufe der Zeit eine natürlichen Veränderung unterliegt, der durch Adaption, also fortgesetztes Trainieren des neuronalen Netzes, auch nach der Lernphase, Rechnung getragen wird.

Eine Weiterbildung besteht darin, daß die Schritte (2) und (3) der Prozessoreinheit eine Endlosschleife bilden.

Eine andere Weiterbildung der Erfindung besteht darin, daß die vorgegebene Abnormalität ein Informationsfluß mit einer Dynamik unterhalb einer vorgegebenen Schranke ist. In diesem Fall kann die Aktion darin bestehen, dem System ein Rauschen zuzuführen. Es ist möglich, das Rauschen anhand eines entsprechenden elektrischen Feldes oder eines entsprechenden magnetischen Feldes zuzuführen. Dabei können sowohl das elektrische Feld als auch das magnetische Feld anhand mindestens einer Elektrode dem System zugeleitet werden.

Eine zusätzliche Weiterbildung besteht darin, daß die vorgegebene Abnormalität einen Informationsfluß von einer Dynamik oberhalb einer vorgegebenen Schranke ist. Darauf kann derart reagiert werden, daß das System mit einem regelmäßigen Signal angeregt wird. Dies kann anhand eines elektrischen oder eines magnetischen Feldes erfolgen. Das elektrische Feld und/oder das magnetische Feld können jeweils anhand mindestens einer Elektrode dem System zugeleitet werden.

Ferner ist es im Rahmen einer anderen Weiterbildung möglich, ein elektrisches und ein magnetisches Feld in Kombination einzusetzen, um der Abnormalität entgegenzuwirken.

Weiterbildungen der-Erfindung ergeben sich auch aus den abhängigen Ansprüchen.

Anhand der folgenden Figuren werden Ausführungsbeispiele der Erfindung näher dargestellt.

Es zeigen

Fig.1 eine Anordnung zur Vorhersage einer Abnormalität eines Systems und zur Durchführung einer der Abnormalität entgegenwirkenden Aktion;

Fig.2 einen Aktor AKT2 als aktive Komponente bestehend aus einem Rechner R, einem Interface IF, einem Energiespeicher BT und zwei Elektroden EL1 und EL2,

Fig.3 Schritte eines Verfahrens zur Durchführung auf einer Prozessoreinheit

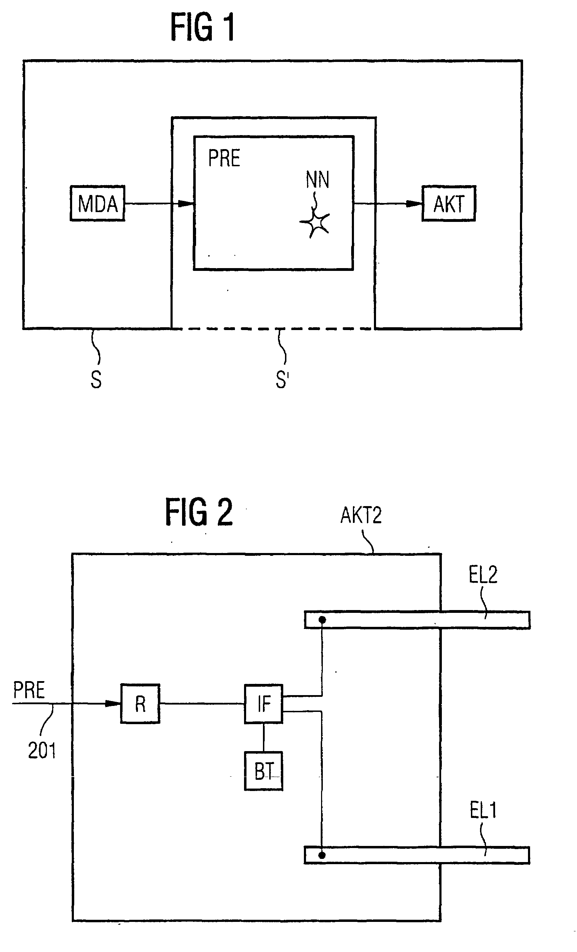

In **Fig.1** ist eine Anordnung zur Vorhersage einer Abnormalität eines Systems und zur Durchführung eines der Abnormalität entgegenwirkenden Aktion dargestellt.

Der Meßdatenaufnehmer MDA zeichnet Meßdaten eines Systems S auf. Vorzugsweise ist der Meßdatenaufnehmer MDA dazu innerhalb des Systems S angeordnet, um vor Ort, die Meßdaten aufzunehmen. Die Meßdaten werden an eine Prozessoreinheit PRE geleitet und dort verarbeitet. Vorzugsweise umfaßt die Prozessoreinheit PRE ein neuronales Netz NN, das nach einem Training weitere von dem Meßdatenaufnehmer MDA aufgezeichnete Meßdaten geeignet auswertet. Bestehen Anzeichen dafür, daß aufgrund der Meßdaten eine Aktion durchzuführen ist, wird ein Aktor AKT von der Prozessoreinheit PRE veranlaßt, eine vorgegebene Aktion durchzuführen. Der Aktor AKT weist vorzugsweise mindestens eine Elektrode auf, die direkt von der Prozessoreinheit PRE angesteuert wird.

Hierbei sei angemerkt, daß die Prozessoreinheit in dem System S' angeordnet ist, wie in Figur 1 anhand der gestrichelten Linie und der zugehörigen Kennzeichnung des Systems S' angedeutet ist.

Das System S umfaßt vorzugsweise den Meßdatenaufnehmer MDA und/oder den Aktor AKT, um jeweils einen direkten Zugriff des Meßdatenaufnehmers MDA auf die Meßdaten und des Aktors AKT auf das System zu gewährleisten.

**Fig.2** zeigt einen anders aufgebauten Aktor AKT 2. Dieser Aktor AKT2 empfängt ebenfalls von der Prozessoreinheit PRE über die Schnittstelle 201 ein Signal, das einem Rechner R, der Teil des Aktors AKT2 ist mitteilt, daß eine vorgegebene Aktion durchgeführt werden soll. In dem Aktor AKT2 ist ferner ein Energiespeicher BT vorgesehen, der von dem Rechner R gesteuert, auf geeignete Art Energie an die Elektroden EL1 und EL2 anlegt. Dabei steuert der Rechner R des Aktors AKT2 die Schnittstelle IF, um vorzugsweise Amplitude und Frequenz der an die Elektroden EL1 und EL2 angelegten Energie zu bestimmen.

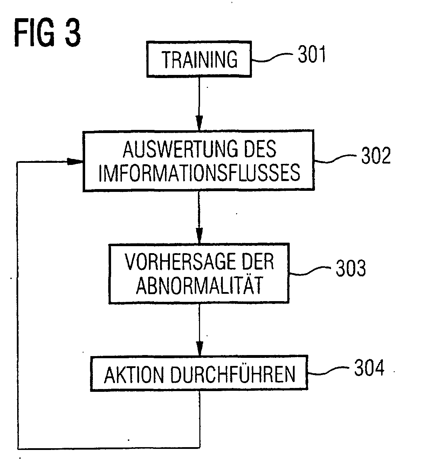

In **Fig.3** sind Schritte des von der Prozessoreinheit PRE durchgeführten Verfahrens dargestellt.

In einem Schritt 301 wird ein neuronales Netz NN trainiert. Hierzu sind Meßdaten von geeignetem Umfang vorgegeben, um nach Abschluß des Trainings eines Aussage darüber machen zu können, ob neue Meßdaten eine Abnormalität des Systems anzeigen. In einem Schritt 302 wird nach Abschluß des Trainings anhand aktueller Meßdaten ein Informationsfluß (siehe [1] oder [2]) ausgewertet. Anhand dieses Informationsflusses kann eine Abnormalität des Systems vor Eintreten dieser Abnormalität angezeigt werden. In einem Schritt 303 wird die Abnormalität vorhergesagt, in einem Schritt 304 eine Aktion durchgeführt, die einem Eintreten der Abnormalität entgegenwirkt. Daraufhin wird vorzugsweise zu dem Schritt 302 gesprungen.

Es folgen zwei Anwendungsbeispiele, die Möglichkeiten einer Vorhersage einer Abnormalität veranschaulichen.

**Anwendung 1: Elektrokardiogramm (EKG) - Daten:**

Eine Anwendung bezieht sich auf die mögliche Vorhersage eines fibrillierenden Herzens. Die Abnormalität besteht darin, daß das Herz nahezu chaotisch schlägt.

Erfindungsgemäß werden EKG-Meßdaten verwendet, um die Dynamik eines Herzens eines Patienten zu erlernen (Trainingsphase des neuronalen Netzes NN). Dabei ist zu bemerken, daß die Dynamik des Herzens in sehr starkem Maß variiert, abhängig beispielsweise von der Tageszeit und von der Art der Tätigkeit, die eine Person gerade durchführt. Dennoch sollen unveränderliche Größen (Vorhersagegröße), die trotz starker Veränderung die Dynamik des Herzens der Person signifikant beschreiben, bestimmt werden. Eine Veränderung der Vorhersagegröße ermöglicht die Vorhersage einer Abnormalität des Herzens. Bei Erkennung der Abnormalität wird ein Kontrollmechanismus in Gang gesetzt, der den normalen Herzrhythmus wiederherstellt.

Die Vorhersagegröße stellt eine Abbildung einer plötzlichen Variation der Komplexität der Dynamik dar, und der Aktor ist in Form einer Elektrode realisiert, die kleine elektrische Impulse dem Herz zuführt.

**Anwendung 2: Elektroencephalogramm (EEG) - Daten:**

Ein anderes dynamisches System ist das Gehirn, vorzugsweise das menschliche Gehirn. Geht man davon aus, daß EEG-Meßdaten Gehirnaktivität repräsentieren, ist es eine Aufgabe, die Signale geeignet zu interpretieren und gegebenenfalls vorgegebene Maßnahmen daran zu knüpfen. So ist ein epileptischer Anfall charakterisiert durch ein synchrones Feuern einer Gruppe von Neuronen, die um einen Mittelpunkt zentriert angeordnet sind. Diese Synchronizität reduziert die Komplexität der Dynamik des Gehirns und wird durch EEG-Meßdaten angezeigt. Im Gegensatz dazu stellt der Normalzustand, also das normal arbeitende Gehirn, einen Zustand aus unregelmäßig feuernden Neuronen dar.

Die Früherkennung eines epileptischen Anfalls wird möglich durch Feststellung einer fortgesetzte Vereinfachung der Dynamik des Gehirns. Der Aktor zur Wiederherstellung des Normalzustandes hat die Aufgabe, dieser Synchronizität, die offensichtlich für den epileptischen Anfall verantwortlich ist, entgegenzuwirken. Dies geschieht vorzugsweise durch Anlegen eines Feldes, wie unten noch vertieft wird.

Nachfolgend wird das zweite Anwendungsbeispiel zur Vermeidung eines epileptischen Anfalls für weitergehende Ausführungen aufgegriffen.

**Die dynamische Vorhersagegröße**

Die Idee besteht in der Erweiterung der statistischen Annäherung gemäß [2] zur Detektion eines Markov-Charakters, der einer gegebenen empirischen Zeitreihe innewohnt. Es ist ein Ziel, einen deterministischen Anteil von einem stochastischen Anteil eines dynamischen Systems im Umfeld der statistischen Testtheorie zu trennen, indem der Informationsfluß des Systems analysiert wird. Die statistische Entwicklung der Dynamik wird getestet gegen eine Hierarchie von Null-Hypothesen, die nichtlinearen Markov-Prozessen mit wachsender Ordnung n entsprechen. Diese Prozesse werden unterteilt in einen deterministischen Anteil und einen stochastischen Anteil auf folgende Weise:

$$x_t = f(x_{t-1}, ..., x_{t-n}) + u \tag{1},$$

wobei u ein additives, nach Gauß verteiltes, Rauschen mit der Varianz $\sigma^2$, $x_t$ ein Meßdatum zur Zeit t und f(..) einen deterministischen Anteil bezeichnen.

Der Markov-Prozeß mit der Ordnung n wird definiert durch dessen bedingte Wahrscheinlichkeitsdichten

$$p\left(x_t \,\middle|\, x_{t-1}, \ldots, x_{t-n}\right) \propto \exp\left(-\frac{\left[x_t - f(x_{t-1}, \ldots, x_{t-n})\right]^2}{2\sigma^2}\right) \tag{2}.$$

Der deterministische Anteil wird implementiert durch ein neuronalen Netzwerks NN, das gemäß dem Maximum-Likelihood-Prinzip [4] angewandt auf die Wahrscheinlichkeitsdichten gemäß Gleichung (2) trainiert wird. Der stochastische Anteil u wird beschrieben durch nach Gauß verteiltes Rauschen, wobei die Varianz $\sigma^2$ bezogen ist auf einen festgelegten mittleren letzten quadratischen Fehler. In anderen Worten, die Null-Hypothesen beinhalten nicht nur die Ordnung des Markov-Prozesses, sondern auch eine tatsächliche deterministische Struktur. Somit, für den Fall daß ein chaotischer Zustand vorliegt, ist die Ordnung der akzeptierten Null-Hypothese die EED (engl.: Effective Embedding Dimension). Dieser Ansatz eröffnet eine Methode zur Bestimmung der EED, während parallel dazu temporäre Meßdaten modelliert werden.

Weiterhin erlaubt dieser Ansatz eine strenge Erweiterung des Konzeptes der ED (engl.: Embedding) für den Fall, daß ein chaotischer Zustand vorherrscht. Die ausdrückliche Bestimmung des deterministischen Anteils ist eine Methode zum Filtern des Rauschens aus der Zeitreihe an.

Die Null-Hypothese wird implementiert durch ein Verfahren beschrieben in [2].

Wie aus [1] und [2] bekannt ist, wird ein Informationsfluß, also ein nichtparametrisches Maß einer vorhersagbaren Entwicklung, benutzt als eine diskriminierende Statistik. Somit wird für jeden vorherzusagenden Zeitpunkt; ein Signifikanztest durchgeführt, wobei die Null-Hypothese (also eine vorgegebene Annahme, die zu überprüfen ist) nur dann akzeptiert wird, wenn der Signifikanztest für alle Größen des vorherzusagenden Zeitpunkts erfüllt wird.

## Analyse menschlicher Epilepsieanfälle

Wie oben beschrieben, stellt eine Anwendung der Erfindung die Analyse von EEG-Meßdaten dar, um einem epileptischen Anfall vorzubeugen. Dabei ist es ein Ziel, zu testen, ob eine dynamische Klassifizierung der Meßdaten für Zeitfenster unterschiedlicher Größe als Vorhersagegrößen benutzt werden können, um einen epileptischen Anfall vorherzusagen. Im besonderen werden zwei Vorhersagegrößen angeführt:

a) Die "Erinnerung" der zugrundeliegenden Dynamik, also die EED (siehe obige Ausführungen);

b) ein nichtparametrisches Maß für eine Vorhersagbarkeit, definiert durch die Integration des Informationsflusses.

Der hier vorgestellte Ansatz setzt nicht voraus, daß die zugrundeliegende Dynamik chaotisch ist (auch wenn sie es sein könnte), sondern legt vielmehr den Schwerpunkt auf einen Zeitraum vor den epileptischen Anfall, um eine auf der Dynamik des Systems basierende Vorhersagegröße für den epileptischen Anfall zu bestimmen.

## Kontrolle des epileptischen Anfalls

Ein epileptischer Anfall kann unterdrückt werden, indem ein konstantes elektrisches Feld den Regionen zugeführt wird, die von dem epileptischen Anfall betroffen sind (siehe [5]).

Gemäß einer Annahme, daß der Normalzustand des Gehirns von chaotischer Dynamik geprägt ist, drückt sich ein epileptischer Anfall durch eine drastische Vereinfachung der Dynamik im Gehirn aus. Dem epileptischen Anfall wird begegnet, indem der Reduktion der Dynamik, also der Synchronizität, wie oben beschrieben, entgegengewirkt wird, indem dem System, hier dem Gehirn, ein Rauschen zugeführt wird.

Die Zuführung dieses Rauschens, wird vorzugsweise durch Anlegen eines elektrischen Feldes oder eines magnetischen Feldes in der direkten Umgebung (möglichst nahe) des Ortes des Geschehens erzeugt. Dazu werden vorzugsweise Elektroden zur Erzeugung eines elektrischen Feldes oder Spulen zur Erzeugung eines magnetischen Feldes verwendet. Durch das elektrische und/oder magnetische Feld werden die beim epileptischen Anfall synchron feuernden Neuronen in deren Synchronizität gestört, es stellt sich wieder ein (scheinbares) chaotisches Feuern der Neuronen im Gehirn ein, der epileptische Anfall ist somit abgewandt.

Es spielt also prinzipiell eine Rolle, daß auf ein abnormales Verhalten eines dynamischen Systems, wobei das abnormale Verhalten mittels einer Vorhersagegröße detektiert wird, geeignet reagiert wird. Diese Reaktion besteht, je nach Anwendungsgebiet, z.B. in der Erzeugung eines chaotischen oder in der Erzeugung eines regelmäßigen Feldes. Diese Aktion, die von dem Aktor durchgeführt wird, hängt von dem jeweiligen Anwendungsgebiet ab. Gemeinsam ist den verschiedenen Varianten des Verfahrens jeweils ein dynamisches Lernen, bei dem eine signifikante Abnormalität einer Vorhersagegröße zugeordnet wird und diese Vorhersagegröße eine Detektion einer sich anbahnenden Abnormalität ermöglicht. Dabei ist es zweckmäßig, innerhalb einer vorgegebenen Zeitdauer vor Eintreten der Abnormalität (des epileptischen Anfalls oder des chaotisch schlagendes Herzens) eine geeignete Aktion mittels eines Aktors durchzuführen. Die Vorhersagegröße ermöglicht also die Erkennung einer Abnormalität, bevor diese wirklich eintritt.

Da sich das gesamte System über einen längeren Zeitraum im Hinblick auf seine dynamisch normale Eigenschaft ändert, ist eines Adaption des ursprünglich gelernten dynamischen Systems zweckmäßig. Wichtig ist es, die Vorhersagegröße zu bestimmen, indem die eine Abnormalität signifikant kennzeichnenden Daten aus dem gesamten dynamischen System in der Vorhersagegröße abgebildet werden. Somit kann auch bei einem starken Schwankungen unterliegenden dynamischen System, z.B. ein Herz, das unterschiedlichsten Belastungen ausgesetzt ist, wobei nicht notwendigerweise eine dieser Belastungen auf eine Abnormalität hinweist, eine Vorhersage der Abnormalität erfolgen.

Im Rahmen dieses Dokuments wurden folgende Veröffentlichungen zitiert:

[1] G. Deco, C. Schittenkopf und B. Schürmann: "Determining the information flow of dynamical systems from continuous probability distributions", Phys. Rev. Lett. 78, Seiten 2345-2348, 1997.

[2] C. Schittenkopf und G. Deco: "Testing non-linear Markovian hypotheses in dynamical systems", Physica D104, Seiten 61-74, 1997.

[3] J. Herz, A. Krogh, R. Pälmer: "Introduction to the Theory of neural computation", Addison-Wesley, 1991.

[4] G. Deco, D. Obradovic: "An Information-Theoretic Approach to Neual Computing", Springer-Verlag, 1996, Kapitel 7.2.

[5] B. Gluckmann, E. Neel, T. Netoff, W. Ditto, M. Spano, S. Schiff: "Electric field suppression of epiletiform activity in hippocampal slices", Journal of Neurophysiology 76, Seiten 4202-4205, 1996.

[6] US 5 251 626 A.

[7] L. Machado et al.: "Sequential Versus Standart Neural networks or Pattern Recognition: An Example Using The Domain of Coronary Heart Disease", Computers in Biology and Medicine, Bd. 27, Nr.4, Juli 1997, Seiten 267-281, XP002097626 UK.

**Patentansprüche**

1. Anordnung zur Vorhersage eines abnormalen Zustands eines dynamischen Systems und zur Durchführung einer dem abnormalen Zustand entgegenwirkenden Aktion unter Verwendung eines Informationsflusses, welcher eine Entwicklung einer Vorhersagbarkeit von mehreren zukünftigen Systemzuständen beschreibt,

   a) bei der ein Meßdatenaufnehmer vorgesehen ist, der in einer Trainingsphase Vergleichsmeßdaten des Systems und in einer Anwendungsphase Versuchsmeßdaten des Systems aufnimmt,
   b) mit einer Prozessoreinheit eingerichtet zur Durchführung folgender Schritte:

      (1) unter Verwendung der Vergleichsmeßdaten wird ein neuronales Netz, welches das System beschreibt, trainiert;
      (2) unter Verwendung des trainierten neuronalen Netzes wird ein Vergleichsinformationsfluß, welcher eine Vergleichsdynamik des Systems beschreibt, bestimmt;
      (3) unter Verwendung der Versuchsmeßdaten wird ein Versuchsinformationsfluß, welcher eine Versuchsdynamik des Systems beschreibt, bestimmt;
      (4) unter Verwendung des Vergleichsinformationsflusses und des Versuchsinformationsflusses wird die Abnormalität als gegeben vorhergesagt dann, wenn sich der Vergleichsinformationsfluß signifikant von dem Versuchsinformationsfluß unterscheidet, und wird die Abnormalität nicht als gegeben vorhergesagt dann, wenn sich der Vergleichsinformationsfluß nicht signifikant von dem Versuchsinformationsfluß unterscheidet;
      (5) ist die Abnormalität des Systems als gegeben vorhergesagt worden, so wird die Aktion durchgeführt;

   c) bei der ein Aktor vorgesehen ist, der die Aktion ausführt.

2. Anordnung nach Anspruch 1,
   bei der die Schritte (2) und (5) der Prozessoreinheit eine Endlosschleife bilden.

3. Anordnung nach Anspruch 1 oder 2,
   bei der die Abnormalität dann als gegeben vorhergesagt wird, wenn Versuchsinformationsfluß signifikant kleiner als der Vergleichsinformationsfluß ist.

4. Anordnung nach Anspruch 3,
   bei der die Aktion darin besteht, das System mit einem chaotischen Signal anzuregen.

5. Anordnung nach Anspruch 4,
   bei der die Aktion darin besteht, dem System ein Rauschen zuzuführen.

6. Anordnung nach Anspruch 5,
   bei der das Rauschen anhand eines entsprechenden elektrischen Feldes zugeführt wird.

7. Anordnung nach Anspruch 6,
   bei der das elektrische Feld anhand mindestens einer Elektrode zugeführt wird.

8. Anordnung nach Anspruch 5,

bei der das Rauschen anhand eines entsprechenden magnetischen Feldes zugeführt wird.

9. Anordnung nach Anspruch 8,
bei der das magnetische Feld anhand mindestens einer Elektrode zugeführt wird.

10. Anordnung nach Anspruch 1 oder 2,
bei der die Abnormalität dann als gegeben vorhergesagt wird, wenn Versuchsinformationsfluß signifikant größer als der Vergleichsinformationsfluß ist.

11. Anordnung nach Anspruch 10,
bei der die Aktion darin besteht, das System mit einem regelmäßigem Signal anzuregen.

12. Anordnung nach Anspruch 11,
bei der das regelmäßige Signal anhand eines elektrischen Feldes zugeführt wird.

13. Anordnung nach Anspruch 12,
bei der das elektrische Feld anhand mindestens einer Elektrode zugeführt wird.

14. Anordnung nach Anspruch 11,
bei der das regelmäßige Signal anhand eines magnetischen Feldes zugeführt wird.

15. Anordnung nach Anspruch 14,
bei der das magnetische Feld anhand mindestens einer Elektrode dem System zugeführt wird.

16. Verfahren zur Vorhersage eines abnormalen Zustands eines dynamischen Systems und zur Durchführung einer dem abnormalen Zustand entgegenwirkenden Aktion unter Verwendung eines Informationsflusses, welcher eine Entwicklung einer Vorhersagbarkeit von mehreren zukünftigen Systemzuständen beschreibt,

    a) bei dem in einer Trainingsphase Vergleichsmeßdaten des Systems und in einer Anwendungsphase Versuchsmeßdaten des Systems gemessen werden,
    b) bei dem unter Verwendung der Vergleichsmeßdaten ein neuronales Netz, welches das System beschreibt, trainiert wird;
    c) bei dem unter Verwendung des neuronalen Netzes ein Vergleichsinformationsfluß, welcher eine Vergleichsdynamik des Systems beschreibt, bestimmt wird;
    d) bei dem unter Verwendung der Versuchsmeßdaten ein Versuchsinformationsfluß, welcher eine Versuchsdynamik des Systems beschreibt, bestimmt wird;
    e) bei dem unter Verwendung des Vergleichsinformationsflusses und des Versuchsinformationsflusses die Abnormalität als gegeben vorhergesagt wird dann, wenn sich der Vergleichsinformationsfluß signifikant von dem Versuchsinformationsfluß unterscheidet, und die Abnormalität nicht als gegeben vorhergesagt wird dann, wenn sich der Vergleichsinformationsfluß nicht signifikant von dem Versuchsinformationsfluß unterscheidet;
    f) bei dem die Aktion durchgeführt wird dann, wenn die Abnormalität des Systems als gegeben vorhergesagt worden ist.

17. Verfahren zur Vorhersage eines abnormalen Zustands eines dynamischen Systems unter Verwendung eines Informationsflusses, welcher eine Entwicklung einer Vorhersagbarkeit von mehreren zukünftigen Systemzuständen beschreibt,

    a) bei dem in einer Trainingsphase Vergleichsmeßdaten des Systems und in einer Anwendungsphase Versuchsmeßdaten des Systems gemessen werden,
    b) bei dem unter Verwendung der Vergleichsmeßdaten ein Vergleichsinformationsfluß, welcher eine Vergleichsdynamik des Systems beschreibt, bestimmt wird;
    c) bei dem unter Verwendung der Versuchsmeßdaten ein Versuchsinformationsfluß, welcher eine Versuchsdynamik des Systems beschreibt, bestimmt wird;
    d) bei dem unter Verwendung des Vergleichsinformationsflusses und des Versuchsinformationsflusses die Abnormalität als gegeben vorhergesagt wird dann, wenn sich der Vergleichsinformationsfluß signifikant von dem Versuchsinformationsfluß unterscheidet, und die Abnormalität nicht als gegeben vorhergesagt wird dann, wenn sich der Vergleichsinformationsfluß nicht signifikant von dem Versuchsinformationsfluß unterscheidet.

**Claims**

1. Arrangement for predicting an abnormal state of a dynamic system and for carrying out an action counteracting the abnormal state by using an information flow which describes a development of a predictability of a plurality of future system states,

   a) in the case of which arrangement a measuring sensor is provided which in a training phase picks up comparison measured data of the system and in an application phase picks up test measured data of the system,
   b) having a processor unit set up for carrying out the following steps:

   (1) a neural network which describes the system is trained by using the comparison measured data;
   (2) a comparison information flow which describes a comparison dynamics of the system is determined by using the trained neural network;
   (3) a test information flow which describes a test dynamics of the system is determined by using the test measured data;
   (4) the abnormality is prescribed as given by using the comparison information flow and the test information flow whenever the comparison information flow differs significantly from the test information flow, and the abnormality is not predicted as given whenever the comparison information flow does not differ significantly from the test information flow;
   (5) the action is carried out if the abnormality of the system has been predicted as given; and

   c) in the case of which arrangement an actuator is provided which executes the action.

2. Arrangement according to Claim 1, in which the steps (2) and (5) of the processor units form an endless loop.

3. Arrangement according to Claim 1 or 2, in which the abnormality is predicted as given whenever the test information flow is significantly smaller than the comparison information flow.

4. Arrangement according to Claim 3, in which the action consists in exciting the system by a chaotic signal.

5. Arrangement according to Claim 4, in which the action consists in feeding the system a noise.

6. Arrangement according to Claim 5, in which the noise is fed with the aid of an appropriate electric field.

7. Arrangement according to Claim 6, in which the electric field is fed with the aid of at least one electrode.

8. Arrangement according to Claim 5, in which the noise is fed with the aid of an appropriate magnetic field.

9. Arrangement according to Claim 8, in which the magnetic field is fed with the aid of at least one electrode.

10. Arrangement according to Claim 1 or 2, in which the abnormality is predicted as given whenever the test information flow is significantly larger than the comparison information flow.

11. Arrangement according to Claim 10, in which the action consists in exciting the system by a regular signal.

12. Arrangement according to Claim 11, in which the regular signal is fed with the aid of an electric field.

13. Arrangement according to Claim 12, in which the electric field is fed with the aid of at least one electrode.

14. Arrangement according to Claim 11, in which the regular signal is fed with the aid of a magnetic field.

15. Arrangement according to Claim 14, in which the magnetic field is fed to the system with the aid of at least one electrode.

16. Method for predicting an abnormal state of a dynamic system and for carrying out an action counteracting the abnormal state by using an information flow which describes a development of a predictability of a plurality of future system states,

a) in the case of which method comparison measured data of the system are measured in a training phase and test measured data of the system are measured in an application phase,
b) in the case of which method a neural network which describes the system is trained by using the comparison measured data;
c) in the case of which method a comparison information flow which describes a comparison dynamics of the system is determined by using the neural network;
d) in the case of which method a test information flow which describes a test dynamics of the system is determined by using the test measured data;
e) in the case of which method the abnormality is predicted as given by using the comparison information flow and the test information flow whenever the comparison information flow differs significantly from the test information flow, and the abnormality is not predicted as given whenever the comparison information flow does not differ significantly from the test information flow; and
f) in the case of which method the action is carried out whenever the abnormality of the system has been predicted as given.

17. Method for predicting an abnormal state of a dynamic system by using an information flow which describes a development of a predictability of a plurality of future system states,

a) in the case of which method comparison measured data of the system are measured in a training phase and test measured data of the system are measured in an application phase,
b) in the case of which method a comparison information flow which describes a comparison dynamics of the system is determined by using the comparison measured data;
c) in the case of which method a test information flow which describes a test dynamics of the system is determined by using the test measured data; and
d) in the case of which method the abnormality is predicted as given by using the comparison information flow and the test information flow whenever the comparison information flow differs significantly from the test information flow, and the abnormality is not predicted as given whenever the comparison information flow does not differ significantly from the test information flow.

**Revendications**

1. Dispositif servant à prédire un état anormal d'un système dynamique et à prendre des mesures permettant de corriger cet état anormal en utilisant un flux d'informations qui décrit un développement d'une prédictibilité de plusieurs états futurs de systèmes,

a) dans lequel est prévu un capteur de données de mesures qui capte des données de mesures comparatives du système lors d'une phase de formation et des données de mesures d'essai du système lors d'une phase de mise en oeuvre,
b) doté d'une unité centrale paramétrée pour l'exécution des étapes suivantes :

(1) un réseau neuronal décrivant le système est formé en utilisant les données de mesures comparatives ;
(2) un flux d'informations comparatif, décrivant une dynamique comparative du système, est défini en utilisant le réseau neuronal formé ;
(3) un flux d'informations d'essai, décrivant une dynamique d'essai du système, est défini en utilisant les données de mesures d'essai ;
(4) l'anomalie est alors indiquée comme étant prédite en utilisant le flux d'informations comparatif et le flux d'informations d'essai, lorsque le flux d'informations comparatif se différencie clairement du flux d'informations d'essai, et l'anomalie n'est alors pas indiquée comme étant prédite lorsque le flux d'informations comparatif ne se différencie pas clairement du flux d'informations d'essai ;
(5) si l'anomalie du système est indiquée comme étant prédite, l'action est exécutée ;

c) dans lequel est prévu un acteur qui exécute l'action.

2. Dispositif selon la revendication 1, dans lequel les étapes (2) et (5) de l'unité centrale forment une boucle sans fin.

3. Dispositif selon la revendication 1 ou 2, dans lequel l'anomalie est indiquée comme étant prédite lorsque le flux d'informations d'essai est clairement plus petit que le flux d'informations comparatif.

**4.** Dispositif selon la revendication 3, dans lequel l'action consiste à démarrer le système avec un signal chaotique.

**5.** Dispositif selon la revendication 4, dans lequel l'action consiste à amener un bruit au système.

**6.** Dispositif selon la revendication 5, dans lequel le bruit est amené à l'aide d'un champ électrique correspondant.

**7.** Dispositif selon la revendication 6, dans lequel le champ électrique est alimenté à l'aide d'au moins une électrode.

**8.** Dispositif selon la revendication 5, dans lequel le bruit est amené à l'aide d'un champ magnétique correspondant.

**9.** Dispositif selon la revendication 8, dans lequel le champ magnétique est alimenté à l'aide d'au moins une électrode.

**10.** Dispositif selon la revendication 1 ou 2, dans lequel l'anomalie est indiquée comme étant prédite lorsque le flux d'informations d'essai est clairement plus grand que le flux d'informations comparatif.

**11.** Dispositif selon la revendication 10, dans lequel l'action consiste à amener un signal régulier au système.

**12.** Dispositif selon la revendication 11, dans lequel le signal régulier est amené à l'aide d'un champ électrique.

**13.** Dispositif selon la revendication 12, dans lequel le champ électrique est alimenté à l'aide d'au moins une électrode.

**14.** Dispositif selon la revendication 11, dans lequel le signal régulier est amené à l'aide d'un champ magnétique.

**15.** Dispositif selon la revendication 14, dans lequel le champ magnétique est amené au système à l'aide d'au moins une électrode.

**16.** Procédé servant à prédire un état anormal d'un système dynamique et à prendre des mesures permettant de corriger cet état anormal en utilisant un flux d'informations qui décrit un développement d'une prédictibilité de plusieurs états futurs de systèmes,

(a) dans lequel des données de mesures comparatives du système sont mesurées lors d'une phase de formation et des données de mesures d'essai du système lors d'une phase de mise en oeuvre,
(b) dans lequel un réseau neuronal décrivant le système est formé en utilisant les données de mesures comparatives ;
(c) dans lequel un flux d'informations comparatif, décrivant une dynamique comparative du système, est défini en utilisant le réseau neuronal ;
(d) dans lequel un flux d'informations d'essai, décrivant une dynamique d'essai du système, est défini en utilisant les données de mesures d'essai ;
(e) dans lequel l'anomalie est alors indiquée comme étant prédite en utilisant le flux d'informations comparatif et le flux d'informations d'essai, lorsque le flux d'informations comparatif se différencie clairement du flux d'informations d'essai, et l'anomalie n'est alors pas indiquée comme étant prédite lorsque le flux d'informations comparatif ne se différencie pas clairement du flux d'informations d'essai ;
(f) dans lequel l'action est exécutée lorsque l'anomalie du système est indiquée comme étant prédite.

**17.** Procédé servant à prédire un état anormal d'un système dynamique en utilisant un flux d'informations qui décrit un développement d'une prédictibilité de plusieurs états futurs de systèmes,

(a) dans lequel des données de mesures comparatives du système sont mesurées lors d'une phase de formation et des données de mesures d'essai du système lors d'une phase de mise en oeuvre,
(b) dans lequel un flux d'informations comparatif, décrivant une dynamique comparative du système, est défini en utilisant les données de mesures comparatives ;
(c) dans lequel un flux d'informations d'essai, décrivant une dynamique d'essai du système, est défini en utilisant les données de mesure d'essai ;
(d) dans lequel l'anomalie est alors indiquée comme étant prédite en utilisant le flux d'informations comparatif et le flux d'informations d'essai, lorsque le flux d'informations comparatif se différencie clairement du flux d'informations d'essai, et l'anomalie n'est alors pas indiquée comme étant prédite lorsque le flux d'informations comparatif ne se différencie pas clairement du flux d'informations d'essai.

## FIG 1

## FIG 2

EP 1 034 464 B1

FIG 3

TRAINING — 301

AUSWERTUNG DES
IMFORMATIONSFLUSSES — 302

VORHERSAGE DER
ABNORMALITÄT — 303

AKTION DURCHFÜHREN — 304

12